# EUROPEAN PATENT SPECIFICATION

(11) **EP 2 913 005 B1**
(45) Date of publication and mention of the grant of the patent: **23.08.2017**
(21) Application number: 14194318.3
(22) Date of filing: 21.11.2014
(51) Int. Cl.: A61B 8/00

(54) **Gel patch for probe and ultrasonic diagnosis apparatus**
Gelpflaster für Sonde und Ultraschalldiagnosevorrichtung
Pastille de gel pour sonde et appareil de diagnostic ultraacoustique

(30) Priority: 28.02.2014 KR 20140024655
(43) Date of publication of application: 02.09.2015
(60) Divisional application: 17178727.8
(73) Proprietor: Samsung Medison Co., Ltd., Gangwon-do 250-870 (KR)
(72) Inventor: Kim, Mi-ri, Gangwon-do (KR); Jin, Gil-ju, Gangwon-do (KR)
(74) Representative: Frey, Sven Holger

(56) References cited:
- US-A- 5 050 436
- US-A- 5 522 878
- US-A1- 2008 281 237

## Description

### RELATED APPLICATION

### BACKGROUND

### 1. Field

One or more embodiments of the present invention relate to gel patches for ultrasonic probes and ultrasonic diagnosis apparatuses including the same.

### 2. Description of the Related Art

In general, an ultrasonic diagnosis apparatus irradiates ultrasound onto an object such as a human or an animal, detects an echo signal reflected from the object, displays a tomographic image of a tissue of the object on a monitor, and provides information necessary for a diagnosis of the object.

The ultrasonic diagnosis apparatus includes an ultrasonic probe for transmitting ultrasound into the object and receiving an echo signal from the object. The ultrasonic diagnosis apparatus including the ultrasonic probe is used to detect foreign substances in a living organism, measure an injury degree, observe a tumor, survey an embryo, etc.

When air exists between the ultrasonic probe and a skin surface of the object, the ultrasound may not be properly transmitted into the object due to attenuation or the like. Thus, a user may apply an ultrasound-transmitting material, such as gel, on the skin surface of the object before bringing the ultrasonic probe into contact with the skin surface of the object. When the ultrasound-transmitting material is applied on the skin surface of the object, the user may feel uncomfortable due to the inherent temperature of the ultrasound-transmitting material and the process of removing the ultrasound-transmitting material from the skin surface of the object after completion of an ultrasonic diagnosis. The invention is defined in claim 1, which is partitioned relative to the document US 2008/0281237 A1.

One or more embodiments of the present invention include gel patches for probes which are attachable/detachable to/from ultrasonic probes, and ultrasonic diagnosis apparatuses including the gel patches.

One or more embodiments of the present invention include gel patches for probes, the gel patches being able to control the temperature of a gel layer.

One or more embodiments of the present invention include gel patches for probes, the gel patches including a supplementary lens that may control a propagation path of ultrasound, and ultrasonic diagnosis apparatuses including the gel patches.

Additional aspects will be set forth in part in the description which follows, and in part, will be apparent from the description, or may be learned by practice of the presented embodiments.

According to one or more embodiments of the present invention, a gel patch for a probe includes: a gel layer that is formed of an ultrasound-transmitting material and prevents formation of a space between an ultrasonic probe and an object; and a supplementary lens that is disposed in the gel layer and controls a propagation path of ultrasound.

The supplementary lens may control at least one of a focal distance and a focal direction of the ultrasound.

The supplementary lens may include at least one of room temperature vulcanizing polymer (RTV) and transparent polymer X (TPX).

The supplementary lens may have at least one of a concave shape and a convex shape.

The gel patch may further include a temperature control layer that controls a temperature of the gel layer.

The temperature control layer may increase or decrease the temperature of the gel layer when oxygen flows into the temperature control layer or when materials contained in the temperature control layer are mixed with each other.

The temperature control layer may include at least one of an exothermic material and an endothermic material.

At least a partial region of the gel layer may be disposed in a region where ultrasound transmitted from the ultrasonic probe propagates.

The temperature control layer may be disposed in a region where the ultrasound transmitted from the ultrasonic probe does not propagate.

The temperature control layer may contact the gel layer.

The temperature control layer may be disposed between the gel layer and the ultrasonic probe.

The gel patch may further include a sheet that contacts the gel layer and the temperature control layer and transfers heat of the temperature control layer to the gel layer.

The sheet may include a thermal conductive material.

An adhesive material, which is attachable/detachable to/from the ultrasonic probe, may be applied on at least a partial region of the sheet.

The adhesive material may include at least one of Micropore, Transpore, Durapore, and Dextrin.

The gel patch may be disposable.

According to one or more embodiments of the present invention, an ultrasonic diagnosis apparatus includes: the above-described gel patch; and an ultrasonic probe that is attachable/detachable to/from the gel patch and transmits/receives ultrasound to/from an object.

### BRIEF DESCRIPTION OF THE DRAWINGS

These and/or other aspects will become apparent and more readily appreciated from the following description of the embodiments, taken in conjunction with the accompanying drawings in which:
FIG. 1 is a partial cutaway perspective view illustrating a schematic internal structure of an ultrasonic probe according to an embodiment of the present invention;
FIG. 2A is a plan view of a gel patch according to an embodiment of the present invention;
FIG. 2B is a cross-sectional view of the gel patch of FIG. 2A;
FIG. 2C is a cross-sectional view illustrating a state in which the gel patch of FIG. 2A is attached to an ultrasonic probe;
FIG. 3 is a cross-sectional view of a gel patch according to another embodiment of the present invention;
FIG. 4 is a table illustrating acoustic characteristic values of tissues of a human body;
FIG. 5A is a cross-sectional view of a gel patch according to another embodiment of the present invention;
FIG. 5B is a cross-sectional view illustrating a state in which the gel patch of FIG. 5A is attached to an ultrasonic probe;
FIG. 6 is a cross-sectional view of a gel patch capable of acquiring a multi-focus, according to another embodiment of the present invention; and
FIG. 7 is a block diagram of an ultrasonic diagnosis apparatus according to an embodiment of the present invention.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, expressions such as "at least one of," when preceding a list of elements, modify the entire list of elements and do not modify the individual elements of the list.

Hereinafter, embodiments of the present invention will be described in detail with reference to the accompanying drawings. In the following description, like reference numerals denote like elements, and redundant descriptions thereof will be omitted.

Throughout the specification, an "object" may include a human, an animal, or a part of a human or animal. For example, the object may include organs such as a liver, heart, womb, brain, breast, abdomen, or the like, or a blood vessel.

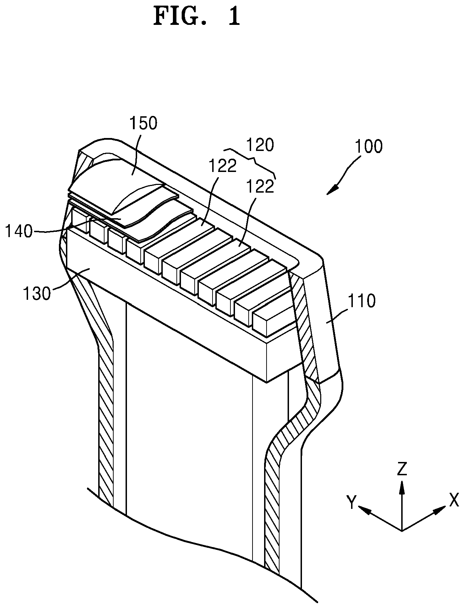

FIG. 1 is a partial cutaway perspective view illustrating a schematic internal structure of an ultrasonic probe 100 according to an embodiment of the present invention.

As illustrated in FIG. 1, the ultrasonic probe 100 according to an embodiment of the present invention may include a housing 110 that forms a body, a transducer 120 that performs transduction between an electrical signal and an acoustic signal, a sound-absorbing layer 130 that absorbs ultrasound transmitted in an opposite direction of an object, a matching layer 140 that matches the acoustic impedance of ultrasound generated by the transducer 120 with the acoustic impedance of the object, and an acoustic lens 150 that focuses ultrasound.

The transducer 120 includes at least one piezoelectric element 122 that vibrates and perform transduction between an electrical signal and an acoustic signal. The piezoelectric element 122 may be formed by dividing a piezoelectric material into a plurality of parts. For example, the piezoelectric element 122 may be formed by dicing a lengthwise long piezoelectric material. However, the embodiments of the present invention are not limited thereto, and a plurality of piezoelectric elements 122 may be formed by pressing a piezoelectric material by using a metal mold. Examples of the piezoelectric material may include a piezoelectric ceramic material, a single-crystal material, or a compound piezoelectric material that is a compound of a high molecular material and the above materials. The transducer 120 may include a piezoelectric micromachined ultrasonic transducer that performs transduction between an ultrasonic signal and an electrical signal according a pressure change, a capacitive micromachined ultrasonic transducer (CMUT) that performs transduction between an ultrasonic signal and an electrical signal according to an electrostatic capacity change, a magnetic micromachined ultrasonic transducer (MMUT) that performs transduction between an ultrasonic signal and an electrical signal according to a magnetic field change, and an optical ultrasonic detector that performs transduction between an ultrasonic signal and an electrical signal according to an optical characteristic change.

The sound-absorbing layer 130 may support the piezoelectric element 122 and may absorb an ultrasonic signal that is transmitted to a backside of the transducer 120 and is not used in a test or diagnosis. A plurality of electrodes may be formed in the sound-absorbing layer 130 to apply a voltage to the transducer 120. Since the electrodes are connected respectively to the piezoelectric elements 122 in the transducer 120, the number of the electrodes may be equal to the number of the piezoelectric elements 122.

The matching layer 140 is disposed on a front surface of the transducer 120. The matching layer 140 gradually changes the acoustic impedance of ultrasound generated by the transducer 120, to match the acoustic impedance of the ultrasound with the acoustic impedance of the object. Herein, the front surface of the transducer 120 may be a surface of the transducer 120 that is adjacent to the object during generation of the ultrasound, and a rear surface of the transducer 120 may be a surface of the transducer 120 that is opposite the front surface of the transducer 120.

The matching layer 140 may be formed to extend along the front surface of the transducer 120. However, the embodiments of the present invention are not limited thereto, and the matching layer 140 may also be formed partially. In addition, the matching layer 140 has a single-layer structure in the present embodiment, but may have a multilayer structure in other embodiments.

The acoustic lens 150 is disposed on the front surface of the transducer 120 to focus the ultrasound generated by the transducer 120. The acoustic lens 150 may be formed of a material such as silicon rubber that has an acoustic impedance similar to that of the object. Also, the acoustic lens 150 may be convex or flat. The acoustic lens 150 may have various shapes according to designs.

An ultrasound-transmitting material may be necessary to prevent formation of a space between the ultrasonic probe 100 and the object when the front side of the ultrasonic probe 100, that is, the acoustic lens 150, contacts the object. The ultrasound-transmitting material may be of a gel type. The ultrasound-transmitting material may be applied on the object or may be applied on the front side of the ultrasonic probe 100. The ultrasound-transmitting material according to an embodiment may be a patch type.

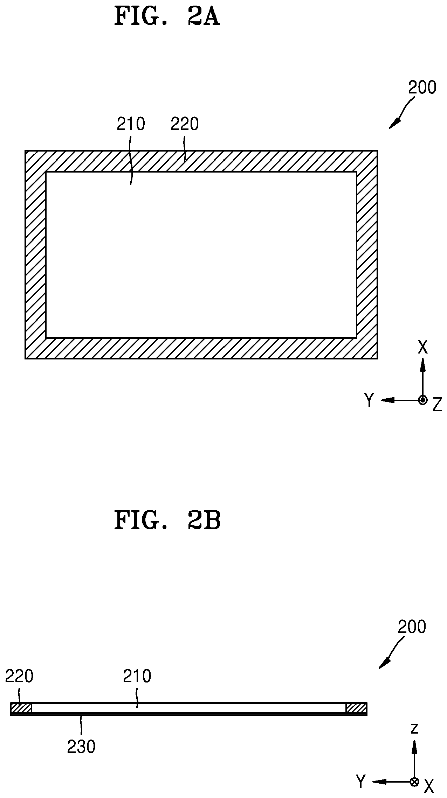

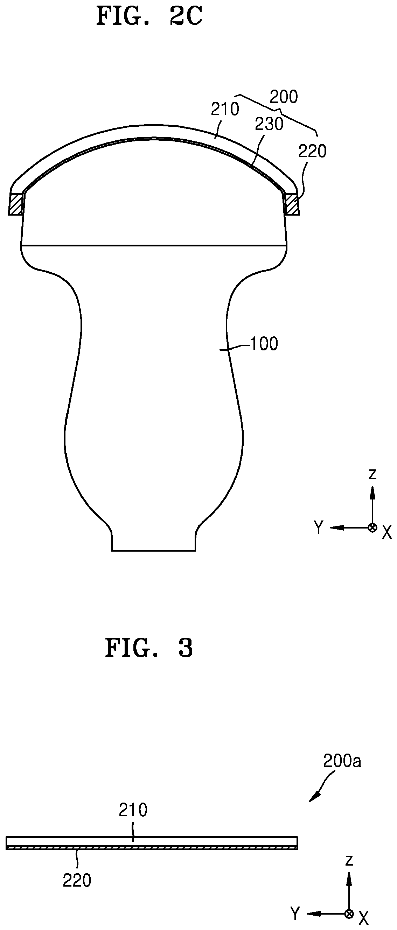

FIG. 2A is a plan view of a gel patch 200 according to an embodiment of the present invention. FIG. 2B is a cross-sectional view of the gel patch 200 of FIG. 2A. FIG. 2C is a cross-sectional view illustrating a state in which the gel patch 200 of FIG. 2A is attached to the ultrasonic probe 100.

As illustrated in FIG. 2A to 2C, the gel patch 200 may be formed of an ultrasound-transmitting material and may include a gel layer 210 that prevents formation of a space between the ultrasonic probe 100 and the object and a temperature control layer 220 that controls the temperature of the gel layer 210. The gel layer 210 may be formed of a material that closely contacts the object to prevent formation of the space between the ultrasonic probe 100 and the object. The acoustic impedance of the gel layer 210 may be substantially equal to the acoustic impedance of the object. The cross-sectional size of the gel layer 210 may be greater than the cross-sectional size of the acoustic lens 150 in the ultrasonic probe 100, and the gel layer 100 may be disposed in a region where the ultrasound transmitted from the ultrasonic probe 100 propagates. Thus, the ultrasound transmitted from the ultrasonic probe 100 may propagate through the gel layer 210 to the object.

The temperature control layer 220 may control the temperature of the gel layer 210. The temperature control layer 220 may contact the gel layer 210 to transfer the heat of the temperature control layer 220 to the gel layer 210. The temperature of the temperature control layer 220 may increase or decrease when oxygen inserted into the temperature control layer 220 or when materials contained in the temperature control layer 220 are mixed with each other. Thus, the heat corresponding to the increased or decreased temperature may be transferred to the gel layer 210 to control the temperature of the gel layer 210.

The temperature control layer 220 may include an exothermic material. For example, the temperature control layer 220 may include alkali metal, alkali earth metal, or sodium acetate that reacts with oxygen to generate heat. As another example, the temperature control layer 220 may include a mixture of potassium permanganate and ethylene glycol that generates heat by a reaction of at least two different materials. The temperature of the temperature control layer 220 including the exothermic material may increase naturally, and the generated heat may be transferred to the gel layer 210 to increase the temperature of the gel layer 210. Thus, when an ultrasonic diagnosis apparatus is used in a cold area or cold weather, user's aversion caused by the inherent cold temperature of the gel layer 210 may be reduced.

The temperature control layer 220 may include an endothermic material. For example, the temperature control layer 220 may include ammonium chloride or ammonium nitrate that reacts with oxygen to absorb heat. As another example, the temperature control layer 220 may include a mixture of ethylene and hydrogen bromide or a mixture of barium hydroxide and ammonium chloride that absorbs heat by a reaction of at least two different materials. The temperature of the temperature control layer 220 including the endothermic material may decrease naturally, and the decreased temperature may be transferred to the gel layer 210 to decrease the temperature of the gel layer 210. Thus, when an ultrasonic diagnosis apparatus is used in a hot area or hot weather, user's aversion caused by the inherent high temperature of the gel layer 210 may be reduced.

The temperature control layer 220 may be disposed to contact the gel layer 210 at an edge of the gel layer 210. For example, the temperature control layer 220 may be disposed in a region where the ultrasound transmitted from the ultrasonic probe 100 does not propagate. As illustrated in FIG. 2C, when the temperature control layer 220 contacts the ultrasonic probe 100, the temperature control layer 220 may be disposed at a side end of the ultrasonic probe 100. Thus, the ultrasound transmitted through the acoustic lens 150 is not attenuated by the temperature control layer 220.

The gel patch 200 according to an embodiment may further include a sheet 230 that contacts the gel layer 210 and the temperature control layer 220. The sheet 230 may be formed of an ultrasound-transmitting material. The sheet 230 may be formed of a material that has a high ultrasound transmittance and may be flexed along the acoustic lens 150 of the ultrasonic probe 100. The gel layer 210 and the temperature control layer 220 may be disposed on a first surface of the sheet 230. A second surface of the sheet 230 may contact the ultrasonic probe 100. Since an adhesive material, which is attachable/detachable to/from the ultrasonic probe 100, is applied on the second surface of the sheet 230, the gel patch 200 may be fixed to the ultrasonic probe 100. The adhesive material may be applied on the entire area of the second surface of the sheet 230. However, the adhesive material may be applied only on a partial area of the second surface of the sheet 230 in order to prevent the adhesive material from remaining on the acoustic lens 150 of the ultrasonic probe 100 due to the attachment/detachment of the gel patch 200. For example, when the gel patch 200 is adhered to the ultrasonic probe 100, the adhesive material may be adhered to a side surface of the ultrasonic probe 100. Examples of the adhesive material may include at least one of Micropore, Transpore, Durapore, and Dextrin.

The sheet 230 may be formed of a material that transmits ultrasound and has thermal conductance. Thus, the temperature of the temperature control layer 220 may be more easily transferred to the gel layer 210.

The gel patch 200 may be disposable. Thus, the gel patch 200 may be attached to the ultrasonic probe 100 whenever an ultrasonic diagnosis is performed, and the gel patch 200 may be disposed upon completion of the ultrasonic diagnosis. Since the gel patch 200 is attached to the ultrasonic probe 100 not to the object, use of the gel patch 200 may be more convenient than use of a liquid gel.

The gel patch 200 may be packed separately. Thus, when the gel patch 200 is unpacked, the materials in the temperature control layer 220 may react with oxygen to control the temperature of the gel layer 210. Also, the user may shake the gel patch 200 after unpacking the gel patch 200. Then, the materials in the temperature control layer 220 are mixed with each other, thereby changing a temperature of the gel layer, and the heat generated by the changed temperature may be transferred to the gel layer 210.

FIG. 3 is a cross-sectional view of a gel patch 200a according to another embodiment of the present invention. The temperature control layer 220 may have a sheet form and be disposed on the bottom surface of the gel layer 210. Then, the adhesive material may be applied on the bottom surface of the temperature control layer 220. Thus, the temperature control layer 220 may control the temperature of the gel layer 210 more rapidly. Also, the temperature control layer 220 may be disposed on the top surface of the gel layer 210, and the adhesive material may be applied on the bottom surface of the gel layer 210.

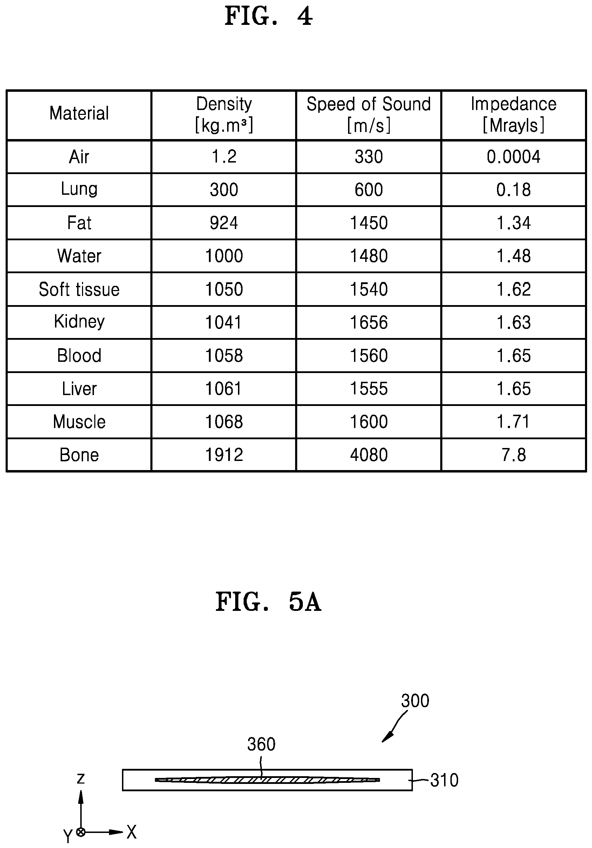

The acoustic characteristics of objects may differ according to tissues in a human body. FIG. 4 is a table illustrating the acoustic characteristic values of tissues of a human body. As illustrated in FIG. 4, the density, sound speed, and sound impedance differ according to tissues. Thus, while the type of the ultrasonic probe 100 may vary according to objects, the type of the ultrasonic probe 100 may be limited in comparison to the variety of acoustic characteristics. For acquisition of a more accurate ultrasonic image, the gel patch 200 according to an embodiment may include a supplementary lens 360 that controls a movement path of the ultrasound.

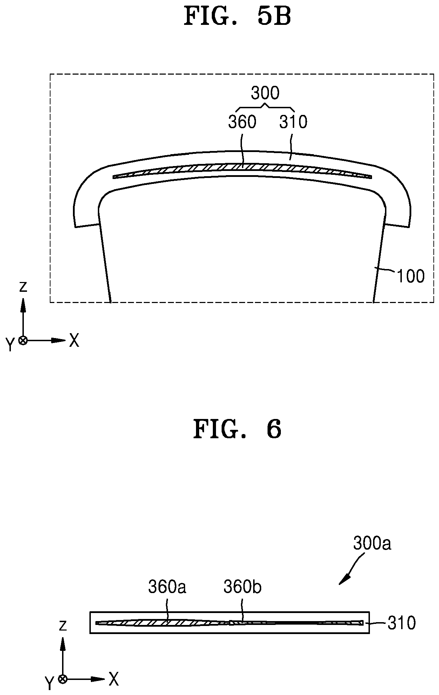

FIG. 5A is a cross-sectional view of a gel patch 300 according to another embodiment of the present invention. FIG. 5B is a cross-sectional view illustrating a state in which the gel patch 300 of FIG. 5A is attached to the ultrasonic probe 300. As illustrated in FIGS. 5A and 5B, the supplementary lens 360 may be disposed in a gel layer 310 to control the movement path of the ultrasound. The supplementary lens 360 may control at least one of a focal distance and a focal direction of the ultrasound. A material of the supplementary lens 360 may have different acoustic characteristics from the material of the gel layer 310. For example, the sound impedance or sound speed of the supplementary lens 360 may be different from those of the gel layer 310. Thus, the supplementary lens 360 may change the movement path of the ultrasound. The supplementary lens 360 may include at least one of room temperature vulcanizing (RTV) and transparent polymer X (TPX). Although the supplementary lens 360 is illustrated as being convex, the embodiments of the present invention are not limited thereto. Thus, the supplementary lens 360 may be concave, and a plurality of supplementary lenses may be provided. An acoustic lens 150 may be disposed in the ultrasonic probe 100 to focus the ultrasound. The acoustic lens 150 in the ultrasonic probe 100 focuses the ultrasound propagating toward the object, and the movement path of the ultrasound may be controlled again by the supplementary lens 360 in the gel patch 300.

When the gel patch 300 is attached to the ultrasonic probe 100, not only the gel layer 310 but also the supplementary lens 360 may be flexed along the surface of the ultrasonic probe 100 as illustrated in FIG. 5B. However, embodiments of the present invention are not limited thereto. In another embodiment, the gel layer 310 may be flexed, but the supplementary lens 360 may not be flexed.

As presented in FIGS. 5A and 5B, the gel patch 300 includes the supplementary lens 360 and the gel layer 310. However, this is merely for convenience of description, and the embodiments of the present invention are not limited thereto. That is, the gel patch 300 may further include the temperature control layer 220 or the sheet 230 described above.

In addition, a plurality of supplementary lenses 360 may be disposed in the gel patch 300. Thus, the ultrasonic probe 100 may acquire a multi-focus ultrasonic image. FIG. 6 is a cross-sectional view of a gel patch 300a capable of acquiring a multi-focus ultrasonic image, according to another embodiment of the present invention. As illustrated in FIG. 6, a convex supplementary lens 360a may be disposed in a partial region of the gel layer 310, and a concave supplementary lens 360b may be disposed in the other region of the gel layer 310. Thus, the ultrasound that passed through the partial region may be focused at a farther distance than the ultrasound that passed through the other region. In addition, the gel patch 300a may include one or more various supplementary lenses.

Since a supplementary lens is disposed in the attachable/detachable gel patch, the user may select a gel patch including a suitable supplementary lens according to the type of an object. Thus, a more accurate ultrasonic image may be acquired by using the selected gel patch.

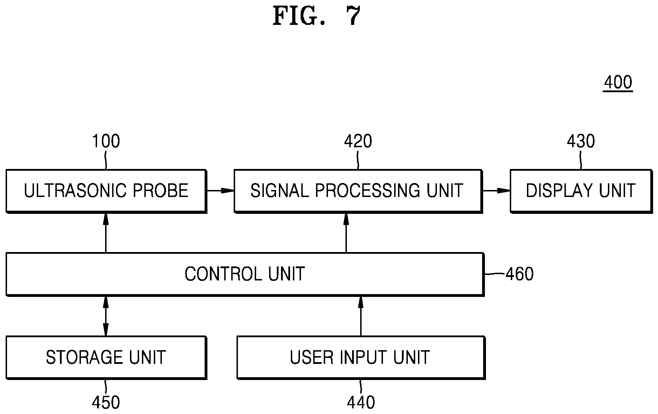

FIG. 7 is a block diagram of an ultrasonic diagnosis apparatus 400 according to an embodiment of the present invention. Referring to FIG. 7, the ultrasonic diagnosis apparatus 400 includes an ultrasonic probe 100 that transmits/receives an ultrasonic signal, a signal processing unit 420 that processes a signal applied to the ultrasonic probe 100 and generates an image, a display unit 430 that displays an image, a user input unit 440 that receives an input of a user command, a storage unit 450 that stores various information, and a control unit 460 that controls overall operations of the ultrasonic diagnosis apparatus 100.

The ultrasonic probe 100 transmits an ultrasonic signal to an object and receives an echo signal reflected from the object, as described above. When the ultrasonic probe 100 is brought into contact with the object, a gel patch 200 may be attached to a front end of the ultrasonic probe 100.

The signal processing unit 420 processes ultrasonic data generated by the ultrasonic probe 100 and generates an ultrasonic image based on the processed ultrasound data. The ultrasonic image may be at least one of a brightness (B) mode image representing a magnitude of an ultrasonic echo signal by brightness, a Doppler mode image representing an image of a moving object in the form of a spectrum by using the Doppler effect, a motion (M) mode image representing a time-dependent motion of an object at a predetermined position, an elasticity mode image representing a difference between a reaction of an object when compression is applied to the object and a reaction of the object when compression is not applied to the object by an image, and a color (C) mode image representing a speed of a moving object by color by using the Doppler effect. The ultrasonic image is generated by a known ultrasonic image generating method, and thus, a detailed description thereof will be omitted. Accordingly, the ultrasonic image according to an embodiment of the present invention may include 1 D, 2D, 3D, and 4D mode images.

The display unit 430 displays information processed by the ultrasonic diagnosis apparatus 400. For example, the display unit 430 may display an ultrasonic image generated by the signal processing unit 420 and may display a graphical user interface (GUI) via which a user input is received.

The display unit 430 may include at least one of a liquid crystal display, a thin film transistor liquid crystal display, an organic light-emitting diode display, a flexible display, a three-dimensional (3D) display, and an electrophoretic display. The ultrasonic diagnosis apparatus 400 may include two or more display units 430 according to embodiments of the present invention.

The user input unit 440 is a unit through which a user inputs data for controlling the ultrasonic diagnosis apparatus 400. The user input unit 440 may include a keypad, a mouse, a touch panel, and a track ball. However, the user input unit 440 is not limited thereto, and may further include other input units such as a jog wheel and a jog switch.

The touch panel may detect a real touch of a pointer to a screen and may also detect a proximity touch of a pointer to a screen when the pointer approaches within a predetermined distance from the screen. In this specification, the pointer refers to a tool for performing a proximity touch or a touch on a predetermined portion of the touch panel. Examples of the pointer may include a stylus pen or a body part such as a finger.

The touch panel may include a touchscreen forming a layer structure with the display unit 430. The touchscreen may be of various types such as a capacitive overlay type, a resistive overlay type, an infrared beam type, an integral strain gauge type, a surface acoustic wave type, and a piezoelectric type. The touchscreen may function not only as the display unit 430 but also as the user input unit 440.

Although not illustrated, various sensors may be provided in or near the touch panel to sense a touch to the touch panel. An example of the sensor for sensing a touch to the touch panel is a tactile sensor. The tactile sensor is a sensor that senses a touch of an object at the degree of human sense or more. The tactile sensor may sense various information values, such as the roughness of a touch surface, the hardness of a touch object, and the temperature of a touch point.

Another example of the sensor for sensing a touch to the touch panel is a proximity sensor. The proximity sensor is a sensor that detects the presence of an object approaching a predetermined detection surface or an object located in the proximity thereof without any mechanical contact, by using an electromagnetic force or infrared rays. Examples of the proximity sensor may include transmission type photoelectric sensors, direct reflection type photoelectric sensors, mirror reflection type photoelectric sensors, high frequency oscillation type proximity sensors, electrostatic capacity type proximity sensors, magnetic type proximity sensors, and infrared proximity sensors.

The storage unit 450 stores various information processed by the ultrasonic diagnosis apparatus 400. For example, the storage unit 450 may store medical data (e.g., images) related to a diagnosis of an object and may also store algorithms or programs to be executed in the ultrasonic diagnosis apparatus 400.

The storage unit 450 may include at least one storage medium from among a flash memory-type storage medium, a hard disk-type storage medium, a multimedia card micro-type storage medium, card-type memories (e.g., an SD card, an XD memory, and the like), Random Access Memory (RAM), Static Random Access Memory (SRAM), Read-Only Memory (ROM), Electrically Erasable Programmable Read-Only Memory (EEPROM), Programmable Read-Only Memory (PROM), a magnetic memory, a magnetic disk, and an optical disk. Also, the ultrasonic diagnosis apparatus 400 may utilize a web storage or a cloud server as the storage unit 450.

The control unit 460 may control overall operations of the ultrasonic diagnosis apparatus 400. That is, the control unit 460 may control an operation of the ultrasonic probe 100, an operation of the signal processing unit 420, and an operation of the display unit 430. For example, by using a user command input through the user input unit 440 or a program stored in the storage unit 450, the control unit 460 may operate such that the signal processing unit 420 generates an image. Also, the control unit 460 may operate such that the image generated by the signal processing unit 420 is displayed on the display unit 430.

As described above, according to the one or more of the above embodiments of the present invention, since the gel layer is attached to the ultrasonic probe, user's inconvenience may be reduced during a diagnosis process. Also, since the temperature of the gel layer is controlled, user's aversion caused by the gel temperature may be reduced. Also, since the supplementary lens is inserted into the gel layer, the transmission/reception of the ultrasound may be improved.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the scope of the present invention as defined by the following claims.

## Claims

1. A gel patch (200) for an ultrasonic probe, comprising:
a gel layer (210) that is formed of an ultrasound-transmitting material and prevents formation of a space between the ultrasonic probe (100) and an object;
a supplementary lens that is disposed in the gel layer and controls a propagation path of ultrasound;
**characterized by**
a temperature control layer (220) that controls a temperature of the gel layer (210) and contacts the gel layer (210), and
wherein the temperature control layer (220) comprises at least one of an exothermic material and an endothermic material.

2. The gel patch (200) of claim 1, wherein the supplementary lens (360) controls at least one of a focal distance and a focal direction of the ultrasound.

3. The gel patch (200) of claim 1 or 2, wherein the supplementary lens (360) comprises room temperature vulcanizing polymer (RTV).

4. The gel patch (200) of any one of claims 1 to 3, wherein the supplementary lens (360) has at least one of a concave shape and a convex shape.

5. The gel patch (200) of any one of claims 1 to 4, wherein the temperature control layer (220) increases or decreases the temperature of the gel layer (210) when oxygen flows into the temperature control layer (220) or when materials contained in the temperature control layer (220) are mixed with each other.

6. The gel patch (200) of any one of claims 1 to 5, wherein at least a partial region of the gel layer (210) is disposed in a region where ultrasound transmitted from the ultrasonic probe (100) propagates.

7. The gel patch (200) of claim 6, wherein the temperature control layer (220) is disposed in a region where the ultrasound transmitted from the ultrasonic probe (100) does not propagate.

8. The gel patch (200) of any one of claims 1 to 7, wherein the temperature control layer (220) is disposed between the gel layer (210) and the ultrasonic probe (100).

9. The gel patch (200) of any one of claims 1 to 8, further comprising a sheet (230) that contacts the gel layer (210) and the temperature control layer (220) and transfers heat of the temperature control layer (220) to the gel layer (210).

10. The gel patch (200) of claim 9, wherein the sheet (230) comprises a thermal conductive material.

11. The gel patch (200) of claim 9 or 10, wherein an adhesive material, which is attachable/detachable to/from the ultrasonic probe (100), is applied on at least a partial region of the sheet (230) wherein preferably the adhesive material comprises Dextrin.

12. The gel patch (200) of claim 1, wherein the gel patch (200) is disposable.

## Patentansprüche

1. Gelpflaster (200) für eine Ultraschallsonde, welches Folgendes aufweiset:
eine Gelschicht (210), die aus einem Ultraschall übertragenden Material gebildet ist und die Bildung eines Raums bzw. Abstands zwischen der Ultraschallsonde (100) und einem Gegenstand verhindert;
eine zusätzliche Linse, die in der Gelschicht angeordnet ist und einen Ausbreitungsweg von Ultraschall steuert;
**gekennzeichnet durch**
eine Temperatursteuerschicht (220), die eine Temperatur der Gelschicht (210) steuert bzw. kontrolliert und die Gelschicht (210) kontaktiert; und
wobei die Temperatursteuerschicht (220) wenigstens eines eines exothermen Materials und eines endothermen Materials aufweist.

2. Gelpflaster (200) nach Anspruch 1, wobei die zusätzliche Linse (360) wenigstens eines eines Fokusabstands und einer Fokusrichtung des Ultraschalls steuert.

3. Gelpflaster (200) nach Anspruch 1 oder 2, wobei die zusätzliche Linse (360) ein bei Raumtemperatur vulkanisierendes Polymer (RTV) aufweist.

4. Gelpflaster (200) nach einem der Ansprüche 1 bis 3, wobei die zusätzliche Linse (360) wenigstens eines einer konkaven Form und einer konvexen Form aufweist.

5. Gelpflaster (200) nach einem der Ansprüche 1 bis 4, wobei die Temperatursteuerschicht (220) die Temperatur der Gelschicht (210) erhöht oder verringert, wenn Sauerstoff in die Temperatursteuerschicht (220) strömt oder wenn in der Temperatursteuerschicht (220) enthaltene Materialien miteinander vermischt werden.

6. Gelpflaster (200) nach einem der Ansprüche 1 bis 5, wobei wenigstens ein Teilbereich der Gelschicht (210) in einem Bereich angeordnet ist, in dem sich von der Ultraschallsonde (100) übertragener Ultraschall ausbreitet.

7. Gelpflaster (200) nach Anspruch 6, wobei die Temperatursteuerschicht (220) in einem Bereich angeordnet ist, in dem sich der von der Ultraschallsonde (100) übertragene Ultraschall nicht ausbreitet.

8. Gelpflaster (200) nach einem der Ansprüche 1 bis 7, wobei die Temperatursteuerschicht (220) zwischen der Gelschicht (210) und der Ultraschallsonde (100) angeordnet ist.

9. Gelpflaster (200) nach einem der Ansprüche 1 bis 8, welches des Weiteren eine Folie (230) aufweist, die die Gelschicht (210) und die Temperatursteuerschicht (220) kontaktiert und Wärme von der Temperatursteuerschicht (220) zu der Gelschicht (210) überträgt.

10. Gelpflaster (200) nach Anspruch 9, wobei die Folie (230) ein thermisch leitendes Material aufweist.

11. Gelpflaster (200) nach Anspruch 9 oder 10, wobei ein adhäsives Material, das an der Ultraschallsonde (100) anbringbar oder von derselben abnehmbar ist, auf wenigstens einem Teilbereich der Folie (230) angeordnet ist, wobei das adhäsive Material vorzugsweise Dextrin aufweist.

12. Gelpflaster (200) nach Anspruch 1, wobei das Gelpflaster (200) ein Einwegartikel ist.

## Revendications

1. Pastille de gel (200) pour une sonde ultrasonique comprenant:
une couche de gel (210) qui est déposée sur un matériau de transmission d'ondes ultrasoniques et qui empêche la formation d'un espace entre la sonde ultrasonique (100) et un objet ;
une lentille supplémentaire qui est disposée dans la couche de gel et commande un chemin de propagation d'ultrasons ;
**caractérisée par**
une couche de contrôle de la température (220) qui commande une température de la couche de gel (210) et qui contacte la couche de gel (210), et
dans laquelle la couche de contrôle de la température (220) comprend au moins un parmi un matériau exothermique et un matériau endothermique.

2. Pastille de gel (200) selon la revendication 1, dans laquelle la lentille supplémentaire (360) commande au moins l'un parmi la distance focale et la direction focale des ultrasons.

3. Pastille de gel (200) selon les revendications 1 ou 2, dans laquelle la lentille supplémentaire (360) comporte un polymère de vulcanisation à la température ambiante (RTV).

4. Pastille de gel (200) selon l'une quelconque des revendications 1 à 3, dans laquelle la lentille supplémentaire (360) comporte au moins une forme concave et une forme convexe.

5. Pastille de gel (200) selon l'une quelconque des revendications 1 à 4, dans laquelle la couche de contrôle de la température (220) augmente ou diminue la température de la couche de gel (210) lorsque de l'oxygène s'écoule dans la couche de contrôle de la température (220) ou lorsque des matériaux contenus dans la couche de contrôle de la température (220) sont mélangés les uns avec les autres.

6. Pastille de gel (200) selon l'une quelconque des revendications 1 à 5, dans laquelle au moins une zone partielle de la couche de gel (210) est disposée dans une zone dans laquelle les ultrasons transmis par la sonde ultrasonique (100) se propagent.

7. Pastille de gel (200) selon la revendication 6, dans laquelle la couche de contrôle de la température (220) est disposée dans une zone dans laquelle les ultrasons transmis par la sonde ultrasonique (100) ne se propagent pas.

8. Pastille de gel (200) selon l'une quelconque des revendications 1 à 7, dans laquelle la couche de contrôle de la température (220) est disposée entre la couche de gel (210) et la sonde ultrasonique (100).

9. Pastille de gel (200) selon l'une quelconque des revendications 1 à 8, comportant en outre un film (230) qui contacte la couche de gel (210) et la couche de contrôle de la température (220), et transfère la chaleur de la couche de contrôle de la température (220) à la couche de gel (210).

10. Pastille de gel (200) selon la revendication 9, dans laquelle le film (230) comporte un matériau thermiquement conducteur.

11. Pastille de gel (200) selon la revendication 9 ou 10, dans laquelle un matériau adhésif qui est fixable/détachable à/de la sonde ultrasonique (100), est appliqué sur au moins une zone partielle du film (230), dans lequel le matériau adhésif comporte de préférence de la Dextrine.

12. Pastille de gel (200) selon la revendication 1, dans laquelle la pastille de gel (200) est jetable.
